(19) Europäisches Patentamt
European Patent Office
Office européen des brevets

(11) Veröffentlichungsnummer: **0 446 717 A2**

(12) **EUROPÄISCHE PATENTANMELDUNG**

(21) Anmeldenummer: **91103071.6**

(22) Anmeldetag: **01.03.91**

(51) Int. Cl.5: **C07D 405/06**, C08F 20/36, C09K 19/38, G02F 1/35, G11B 7/24, C09K 9/02

(30) Priorität: **10.03.90 DE 4007636**

(43) Veröffentlichungstag der Anmeldung: **18.09.91 Patentblatt 91/38**

(84) Benannte Vertragsstaaten: **BE DE FR GB IT NL**

(71) Anmelder: **BASF Aktiengesellschaft**
**Carl-Bosch-Strasse 38**
**W-6700 Ludwigshafen(DE)**

(72) Erfinder: **Wagenblast, Gerhard, Dr.**
**Bachweg 8**
**W-6719 Weisenheim(DE)**
Erfinder: **Bach, Volker, Dr.**
**Robert-Schuman-Strasse 8**
**W-6730 Neustadt(DE)**
Erfinder: **Cabrera, Ivan, Dr.**
**Am Herrmannsberg 21**
**W-6080 Gross-Gerau(DE)**
Erfinder: **Dittrich, Achim**
**Kreuzweg 19**
**W-6222 Geisenheim(DE)**
Erfinder: **Ringsdorf, Helmut, Prof.Dr.**
**Kehlweg 41**
**W-6500 Meinz-Gonsenheim(DE)**

(54) **Photochrome Monomere und hieraus hergestellte photochrome Polymerisate.**

(57) Monomere der allgemeinen Formel I

$$CH_2{=}\overset{\displaystyle R}{\overset{|}{C}}{-}X{-}A{-}F \qquad (I)$$

worin die Variablen die folgende Bedeutung haben:

- R Wasserstoff- oder Chloratom oder Methylgruppe;
- X zweiwertige verknüpfende funktionelle Gruppe oder Kohlenstoff-Kohlenstoff-Einfachbindung;
- A flexible abstandshaltende langkettige Gruppe;
- F über das Stickstoffatom mit A verbundene Fulgidimidgruppe;

dienen der Herstellung neuer photochromer und neuer photochromer flüssigkristalliner Polymerisate I, welche mindestens eine Gruppe der allgemeinen Formel IV

X-A-F (IV)

enthalten, worin die Variablen X, A und F die vorstehend angegebenen Bedeutungen haben. Sowohl die neuen Monomeren I als auch die neuen Polymerisate I können in der Holographie, der laseroptischen Datenaufzeichnung, der Reprographie, der nichtlinearen Optik und der Anzeige von Informationen verwendet werden.

EP 0 446 717 A2

Die vorliegende Erfindung betrifft neue photochrome Monomere der allgemeinen Formel I

$$CH_2{=}\overset{\overset{\displaystyle R}{|}}{C}{-}X{-}A{-}F \qquad (I)$$

worin die variablen die folgende Bedeutung haben:

- R Wasserstoff- oder Chloratom oder Methylgruppe;
- X zweiwertige verknüpfende funktionelle Gruppe oder Kohlenstoff-Kohlenstoff-Einfachbindung;
- A flexible abstandshaltende langkettige Gruppe;
- F über das Stickstoffatom mit A verbundene Fulgidimidgruppe.

Außerdem betrifft die vorliegende Erfindung neue photochrome und photochrome flüssigkristalline Polymerisate I, welche unter Verwendung der neuen photochromen Monomeren I hergestellt werden. Des weiteren betrifft die vorliegende Erfindung die Verwendung der neuen photochromen Monomeren I und der neuen photochromen und photochromen flüssigkristallinen Polymerisate I in der Holographie, der laseroptischen Datenaufzeichnung, der Reprographie, der nichtlinearen Optik und der Anzeige von Informationen.

Als Fulgide (von lat. fulgere = glänzen, strahlen) werden meist intensiv gefärbte Dialkylidenbernsteinsäureanhydrid-Derivate bezeichnet, welche häufig Photochromie zeigen. D.h., daß die Fulgide und die Fulgidimide, beim Bestrahlen mit ultraviolettem Licht aus ihrer Dialkylidenbernsteinsäureanhydrid-Form (nicht-cyclische Form) durch Bestrahlen mit ultraviolettem Licht in ihre intensiv gefärbte Dihydrobenzo-Form (cyclische Form) umgewandelt werden können, wonach die cyclische Form der Fulgide oder Fulgidimide durch Bestrahlen mit sichtbarem Licht wieder in die farblose oder nur schwach gefärbte nicht-cyclische Form zurückverwandelt werden kann.

Niedermolekulare Fulgide und Fulgidimide, welche ein ausgeprägtes photochromes Verhalten zeigen, sind aus der US-A-4 220 708, der DE-A-25 32 224, der DE-A-26 47 850, dem Artikel von Akira Kaneko et al., "Photochemical Fatigue Resistances and Thermal Stabilities of Heterocyclic Fulgides in PMMA Film", in Bulletin of the Chemical Society of Japan, Band 61, Seiten 3569 bis 3573, 1988, und dem Artikel von Veerle Deblauwe et al., "Quantum yields of the photochromic reactions of heterocyclic fulgides and fulgimides", in Makromolekulare Chemie, Band 189, Seiten 2503 bis 2512, 1988, bekannt.

Die Verwendung dieser niedermolekularen Fulgide und Fulgidimide in Vorrichtungen für die Speicherung von Informationen, für die holographische Aufzeichnung, für die Anfertigung von Telekopien oder für die Anzeige von Informationen geht gleichfalls aus der US-A 4 220 708, DE-A 25 32 224 und DE-A 26 47 850 hervor.

Des weiteren ist aus der JP-A-01/26846 ein N-Stearylfulgidimid bekannt, welches sich für die Herstellung von Langmuir-Blodgett-Filmen eignet.

Darüber hinaus geht aus der EP-A 0 070 631 die Stabilisierung von Fulgiden und Fulgidimiden durch ihre Einlagerung in Tonmineralien wie Bentonit oder Montmorillonit hervor. Diese stabilisierten Zusammensetzungen können als Papierbeschichtungsmittel oder als Füllmittel für Kunststoffe verwendet werden.

Ferner ist aus dem Artikel von Yukaei Hattori et al., "Photochromism in anisotropic matrices", in dem Tagungsband des International Symposium on Chemistry of Functional Dyestuffs, Osaka, Japan, Juni 1988, P-84, ein lichtempfindliches Aufzeichnungselement oder eine laseroptische Datenplatte bekannt, worin Fulgide in anisotropen Matrices wie nematisch flüssigkristalline Phasen, gestreckte Polyvinylalkoholfilme oder gestreckte Polyethylenfilme vorliegen. Mit Hilfe dieser lichtempfindlichen Aufzeichnungselemente oder laseroptischen Datenplatten soll verhindert werden, daß die eingeschriebene Information beim Lesen wieder gelöscht wird, was häufig bei Aufzeichnungselementen und laseroptischen Datenplatten auf der Basis photochromer Substanzen der Fall ist.

Die bislang bekannten niedermolekularen Fulgide und Fulgidimide weisen bei ihrer Verwendung in der Holographie, der laseroptischen Datenaufzeichnung, der Reprographie, der nichtlinearen Optik und der Anzeige von Informationen Nachteile auf. So erfüllen die Vorrichtungen für die Holographie, die laseroptischen Datenplatten, die lichtempfindlichen Aufzeichnungselemente für die Reprographie, die Vorrichtungen für die nichtlineare Optik und die Elemente zur Anzeige von Informationen bei weitem nicht alle Ansprüche und Erwartungen, welche heutzutage an solche Vorrichtungen in der Praxis gestellt werden. Häufig lassen sich die bislang bekannten niedermolekularen Fulgide oder Fulgidimide nur sehr schlecht in dünnen Schichten orientieren. Sofern sie sich orientieren lassen, sind die betreffenden Schichten häufig instabil, was letztlich zu einem Verlust der in diese Schichten eingeschriebenen Informationen führt. Häufig zeigen diese Schichten auch ein rasches "Ermüden" der Photochromie, was seine Ursache in dem vermehrten Auftreten von Nebenreaktionen bei der wiederholten Umwandlung der Fulgide und Fulgidimide aus ihrer

nicht-cyclischen in ihre cyclische Form und zurück hat. Darüber hinaus sind Schichten aus den bekannten niedermolekularen Fulgiden und Fulgidimiden nicht mechanisch stabil, und die in diese Schichten eingeschriebenen Informationen können u.U. auch durch Diffusionsvorgänge wieder zerstört werden.

Es ist daher in hohem Maße wünschenswert, neue photochrome Materialien zu finden, welche nicht nur thermisch und photochemisch, sondern auch mechanisch stabil sind und in welchen Diffusionsvorgänge keine Rolle mehr spielen.

Polymerisate mit mindestens einer mesogene Seitengruppe, welche eine nematisch oder smektisch flüssigkristalline Phase zu bilden vermag, sowie die Verwendung dieser Polymerisate in der Holographie, der laseroptischen Datenaufzeichnung und der Anzeige von Informationen sind beispielsweise aus den Patentschriften EP-A-0 188 785, DE-A-27 22 589, EP-A-0 231 858 (DE-A-36 03 267), DE-A-36 23 395 (GB-A-2 193 338) oder der EP-A-0 231 857 (DE-A-36 03 268) bekannt. Wie aus der Patentschrift EP-A-0 171 045 (DE-A-34 29 438) hervorgeht, können diese Polymerisate auch noch pleiochroitische Seitengruppen enthalten.

Polymerisate mit mindestens einer mesogenen Seitengruppe, welche eine cholesterisch flüssigkristalline Phase zu bilden vermag, sowie deren Verwendung in der laseroptischen Datenaufzeichnung sind aus der DE-A-36 04 757 bekannt.

Polymerisate mit mindestens einer mesogenen Seitengruppe, welche eine ferroelektrische smektisch flüssigkristalline Phase zu bilden vermag, sowie ihre Verwendung in der laseroptischen Datenaufzeichnung, der Holographie und der Anzeige von Informationen sind aus den Patentschriften DE-A-38 23 153, DE-A-38 23 154, EP-A-0 184 482, EP-A-0 228 703, EP-A-0 258 898, EP-A-0 271 900 oder EP-A-0 274 128 bekannt.

Polymerisate mit mindestens einer Seitengruppe, welche nicht-zentrosymmetrisch ist und ein leicht polarisierbares konjugiertes $\pi$-Elektronensystem sowie mindestens eine endständige Elektronenakzeptorgruppe aufweist, sowie deren Verwendung in der nichtlinearen Optik gehen aus den Patentschriften US-A-4 755 574, WO-A-88/04305, EP-A-0 271 730, EP-A-0 235 506, US-A-4 694 066 oder US-A-4 757 130 hervor.

Polymerisate mit mindestens einem geradkettigen $C_{10}$- bis $C_{20}$-Alkylrest als Seitengruppe sind aus der EP-A-0 232 113 bekannt. Diese Polymerisate können für die laseroptische Datenaufzeichnung verwendet werden.

Aus dem Artikel von Michael M. Carpenter et al., "The characterization of Langmuir-Blodgett films of a non-linear optical, side-chain liquid crystalline polymer", in Thin Solid Films, Band 161 (1988), Seiten 315 bis 324, sind Polymerisate bekannt, welche sowohl

i) mindestens eine mesogene Gruppe, welche eine ferroelektrische smektisch flüssigkristalline Phase zu bilden vermag, als auch

ii) mindestens eine nicht-zentrosymmetrische, ein leicht polarisierbares konjugiertes $\pi$-Elektronensystem sowie mindestens eine endständige Elektronenakzeptorgruppe enthaltende Gruppe

als Seitengruppen aufweisen. Diese Polymerisate werden zur Herstellung von Langmuir-Blodgett-Schichten verwendet.

Polymerisate der vorstehend genannten Art, welche durch (Co)Polymerisation von ethylenisch ungesättigte Gruppen enthaltenden Monomeren herstellbar sind, sind beispielsweise aus den Patentschriften DE-A-38 23 153, DE-A-38 23 154, EP-A-0 184 482, EP-A-0 228 703, EP-A-0 271 900, DE-A-36 04 757, EP-A-0 188 785, EP-A-0 231 858, DE-A-36 23 395 (GB-A-2 193 338), EP-A-0 231 857 (DE-A-36 03 268), EP-A-0 171 045 (DE-A-34 29 438), US-A-4 755 574, WO-A-88/04305 oder EP-A-0 271 730 oder dem vorstehend aufgeführten Artikel von Michael M. Carpenter in Thin Solid Films, Band 161 (1988), Seiten 315 bis 324 bekannt.

Darüber hinaus sind aus den Patentschriften DE-A-33 34 056, DE-A-0 141 512, US-A-4 762 912, US-A-4 358 391, US-A-4 388 453 und US-A-4 410 570 Polymerisate bekannt, welche durch Addition mindestens eines mindestens eine ethylenisch ungesättigte Gruppe enthaltenden Monomeren an Organopolysiloxane mit mindestens einer SiH-Gruppe herstellbar sind. Auch diese bekannten Polymerisate können für die laseroptische Datenaufzeichnung, in der Holographie oder in der nichtlinearen Optik angewandt werden.

Bekanntermaßen haben diese Polymerisate gegenüber den ihren Seitengruppen entsprechenden niedermolekularen Verbindungen Vorteile. Diese liegen insbesondere darin, daß die anwendungstechnischen Eigenschaften von Vorrichtungen für die Holographie, laseroptischen Datenplatten, lichtempfindlichen Aufzeichnungselementen für die Reprographie, Vorrichtungen für die nichtlineare Optik und Elementen zur Anzeige von Informationen auf der Basis dieser bekannten Polymerisate nicht mehr durch Diffusions- und/oder Kristallisationsvorgänge verschlechtert werden. Darüber hinaus sind dünne Schichten aus diesen bekannten Polymerisaten im allgemeinen mechanisch stabiler als Schichten aus den niedermolekularen Verbindungen, welche den Seitengruppen der bekannten Polymerisate entsprechen.

Indes weisen die bekannten Polymerisate den schwerwiegenden Nachteil auf, daß sie keine photochromen Eigenschaften haben, und die bislang bekannten niedermolekularen Fulgide haben den Nachteil, daß

sie sich nicht für die Herstellung von Polymerisaten eignen.

Aufgabe der vorliegenden Erfindung ist es, neue Monomere bereitzustellen, welche in einfacher Weise erhältlich sind, photochrome Eigenschaften aufweisen und sich für die Herstellung von neuen Polymerisaten des unterschiedlichsten Aufbaus mit photochromen Eigenschaften eignen. Außerdem sollen sich sowohl die neuen photochromen Monomeren als auch die mit ihrer Hilfe hergestellten neuen photochromen Polymerisate für die Herstellung von Langmuir-Blodgett-Schichten, Vorrichtungen für die Holographie, laseroptischen Datenplatten, lichtempfindlichen Aufzeichnungselementen für die Reprographie, Vorrichtungen für die nichtlineare Optik und Elemente zur Anzeige von Informationen eignen.

Überraschenderweise konnte diese Aufgabe durch die eingangs definierten neuen photochromen Monomeren der allgemeinen Formel I gelöst werden. Hierbei war es im Hinblick auf den Stand der Technik überraschend, daß diese neuen photochromen Monomeren I besonders gut für die Herstellung der neuen photochromen Polymerisate I geeignet sind.

Demnach handelt es sich bei dem Gegenstand der vorliegenden Erfindung um die Eingangs definierten neuen photochromen Monomeren der allgemeinen Formel I, welche im folgenden der Kürze halber als "erfindungsgemäße Monomeren I" bezeichnet werden.

Die erfindungsgemäßen Monomeren I werden durch die allgemeine Formel I beschrieben.

In der allgemeinen Formel I bezeichnet die Variable R entweder ein Wasserstoff- oder ein Chloratom oder eine Methylgruppe. Erfindungsgemäß ist es von Vorteil, wenn die Variable R ein Wasserstoffatom oder eine Methylgruppe bezeichnet.

In der allgemeinen Formel I bezeichnet die Variable X entweder eine zweiwertige verknüpfende funktionelle Gruppe oder eine Kohlenstoff-Kohlenstoff-Einfachbindung.

Beispiele erfindungsgemäß geeigneter zweiwertiger verknüpfender funktioneller Gruppen sind das Sauerstoffatom, das Schwefelatom, die Imidgruppe ($-NR^1-$), worin $R^1$ für ein Wasserstoffatom oder eine $C_1$- bis $C_4$-Alkylgruppe steht, die Estergruppe

Estergruppe

$$(-\underset{\underset{O}{\|}}{C}-O- \quad \text{oder} \quad -O-\underset{\underset{O}{\|}}{C}-)\ ,$$

Thioestergruppe

$$(-\underset{\underset{O}{\|}}{C}-S- \quad \text{oder} \quad -S-\underset{\underset{O}{\|}}{C}-)\ ,$$

Amidgruppe

$$(-\underset{\underset{O}{\|}}{C}-NR^1- \quad \text{oder} \quad -NR^1-\underset{\underset{O}{\|}}{C}-)\ ,$$

worin $R^1$ die vorstehend angegebene Bedeutung hat, oder

Urethangruppe

$$(-O-\underset{\underset{O}{\|}}{C}-NR^1- \quad \text{oder} \quad -NR^1-\underset{\underset{O}{\|}}{C}-O-)\ ,$$

worin $R^1$ die vorstehend angegebene Bedeutung hat.

Beispiele geeigneter Reste $R^1$, welche eine $C_1$- bis $C_4$-Alkylgruppe darstellen, sind Methyl, Ethyl, Propyl und n-Butyl.

Erfindungsgemäß ist es von besonderem Vorteil, wenn die Variable X eine Estergruppe

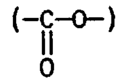

ist,
welche die olefinisch ungesättigte Gruppe des erfindungsgemäßen Monomeren I mit A in der folgenden Weise verknüpft:

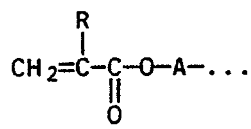

In der allgemeinen Formel I bezeichnet die Variable A eine flexible abstandshaltende langkettige Gruppe.

Beispiele erfindungsgemäß geeigneter flexibler abstandshaltender langkettiger Gruppen A sind diejenigen der allgemeinen Formel II

$$-(CH_2)_k- \qquad (II)$$

worin der Index k eine ganze Zahl von 1 bis 20, vorzugsweise 2 bis 15 und insbesondere 3 bis 10 bedeutet. Erfindungsgemäß ist es von besonderem Vorteil, wenn k für 6 steht.

Erfindungsgemäß ist es von ganz besonderem Vorteil, wenn die Variable R für ein Wasserstoffatom oder eine Methylgruppe steht, die Variable X eine Estergruppe

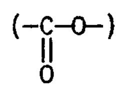

bezeichnet, welche die olefinisch ungesättigte Gruppe des erfindungsgemäßen Monomeren I mit A in der vorstehend aufgezeigten vorteilhaften Weise verknüpft, und die flexible abstandshaltende langkettige Gruppe A eine Hexan-1,6-diyl-gruppe ist.

In der allgemeinen Formel I bedeutet die Variable F ein über das Stickstoffatom mit A verbundene Fulgidimidgruppe.

Als struktureller und erfindungswesentlicher Bestandteil F der erfindungsgemäßen Monomeren I kommen all diejenigen Fulgidimidgruppen in Betracht, welche - ähnlich wie die niedermolekularen Fulgidimide, von denen sie sich ableiten - das für die Fulgide typische, mit Hilfe des nachfolgenden Schemas 1 verdeutlichte photochrome Verhalten zeigen:

Schema 1

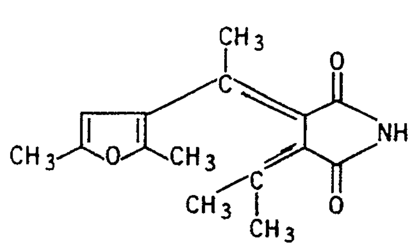

nicht-cyclische Form
schwach gefärbt oder
farblos

ultraviolettes Licht

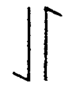

sichtbares Licht

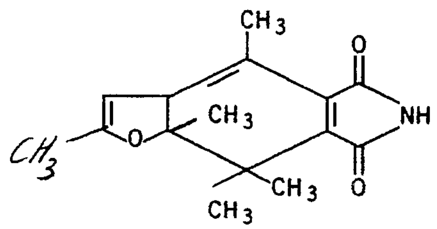

cyclische Form
farbig

Beispiele erfindungsgemäß gut geeigneter Fulgidimid-Gruppen sind:

F1

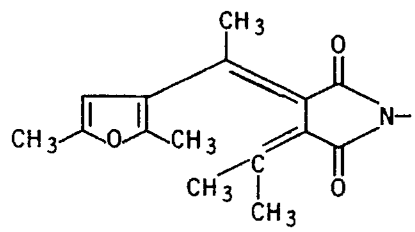

und die entsprechende cyclische Form

F2

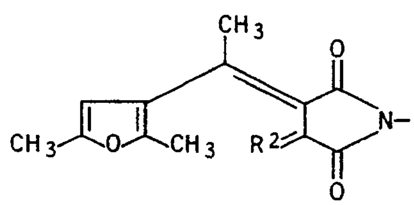

und die entsprechende cyclische Form,

worin $R^2$ einen Adamant-2-yliden-Rest bezeichnet;

F3

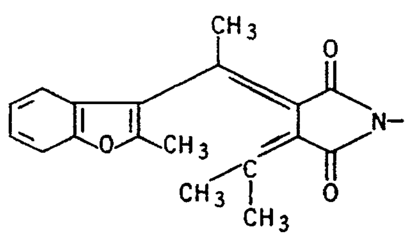

und die entsprechende cyclische Form,

F4

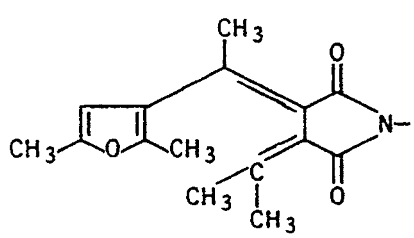

und die entsprechende cyclische Form.

Die erfindungsgemäß gut geeigneten Fulgidimide F1 bis F4 leiten sich von niedermolekularen Fulgidimiden ab, welche aus der US-A-4 220 708, der DE-A-26 47 850 oder der DE-A-25 32 224 bekannt sind. Weitere gut geeignete bekannte Fulgidimide, welche als Fulgidimidgruppen F in den erfindungsgemäßen Monomeren I in Betracht kommen, können diesen Patentschriften entommen werden.

Von den vorstehend beispielhaft aufgeführten Fulgidimidgruppen F1 bis F4 ist die Fulgidimidgruppe F1 von besonderem Vorteil und wird deshalb erfindungsgemäß ganz besonders bevorzugt verwendet.

Beispiele für vorteilhafte erfindungsgemäße Monomere I sind demnach:

MI-1

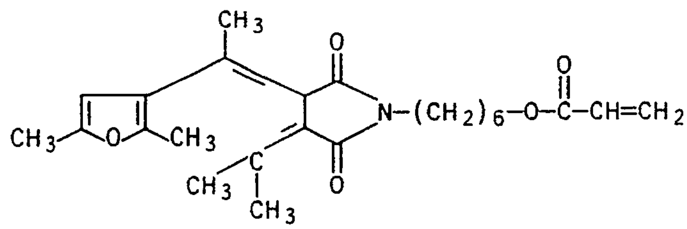

und die entsprechende cyclische Form;

MI-2

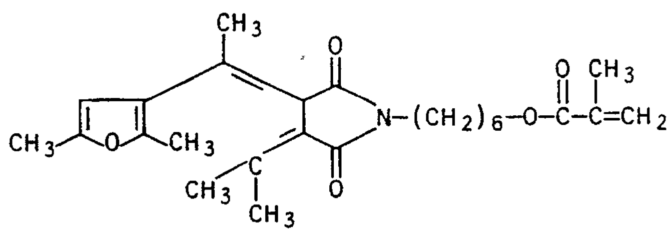

und die entsprechende cyclische Form;

MI-3

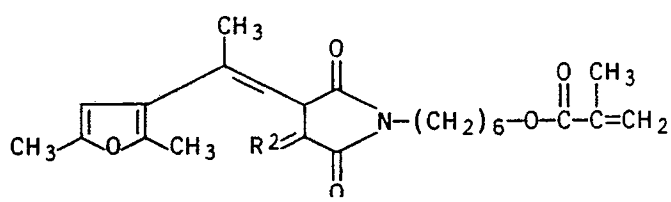 ,

worin $R^2$ einen Adamant-2-yliden-Rest bezeichnet, und die entsprechende cyclische Form.

Von diesen erfindungsgemäßen Monomeren MI-1 bis MI-3 sind MI-1 und MI-2 ganz besonders vorteilhaft.

Methodisch gesehen weist die Herstellung der erfindungsgemäßen Monomeren I keine Besonderheiten auf, sondern es werden hierbei Synthesemethoden verwendet, welche auf dem Gebiet der präparativen organischen Chemie üblich und bekannt sind.

Das Herstellverfahren geht aus von einem Fulgid, d.h. dem Dialkylidenbernsteinsäureanhydrid-Derivat, dessen Anhydridring mit Ammoniak unter Bildung der entsprechenden Amidsäure gespalten wird. Anschließend wird die freie Carboxylgruppe der betreffenden Amidsäure mit Diazomethan verestert. Der hierbei resultierende Amidester wird dann unter Abspaltung von Methanol zu dem betreffenden Fulgidimid kondensiert. Dieses Fulgidimid wird anschließend mit einer Verbindung der Formel III

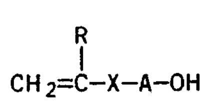

(III)

in der Gegenwart einer, bezogen auf das Fulgidimid, äquimolaren Menge an Triarylphosphin und einer katalytischen Menge eines Diazodicarbonsäuredialkylesters umgesetzt, wonach man das hieraus resultierende betreffende erfindungsgemäße Monomer I aus dem Reaktionsgemisch isoliert.

Hierbei haben in der allgemeinen Formel III die Variablen R, X und A die vorstehend angegebenen Bedeutungen.

Beispiele geeigneter Triarylphosphine sind Triphenylphosphin und die methylierten Triphenylphosphine, von denen Triphenylphosphin bevorzugt verwendet wird.

Beispiele geeigneter Diazodicarbonsäuredialkylester sind der Dimethyl-, der Diethyl-, der Di-n-propyl- und der Di-n-butylester.

Die erfindungsgemäßen Monomeren I haben vorzügliche photochrome Eigenschaften und können deshalb auf allen Anwendungsgebieten in allen Verwendungszwecken die bislang bekannten photochromen Materialien in vollem Umfang ersetzen. So können sie in der Holographie, der laseroptischen Datenaufzeichnung, der Reprographie, der nichtlinearen Optik oder der Anzeige von Informationen verwendet werden, und die Vorrichtungen für die Holographie, die laseroptischen Datenplatten, die lichtempfindlichen Aufzeichnungselemente für die Reprographie, die Vorrichtungen für die nichtlineare Optik und die Elemente zur Anzeige von Informationen auf der Basis der erfindungsgemäßen Monomeren I weisen zahlreiche anwendungstechnische Vorteile gegenüber dem Stand der Technik auf. Vor allem aber eignen sich die erfindungsgemäßen Monomere I für die Herstellung photochromer und photochromer flüssigkristalliner Polymerisate. Die aus den oder mit Hilfe der erfindungsgemäßen Monomeren I hergestellten neuen photochromen und photochromen flüssigkristallinen Polymerisate werden im folgenden als "erfindungsgemäße Polymerisate I" bezeichnet.

Bei den erfindungsgemäßen Polymerisaten I kann es sich um ein beliebiges Polymeres, beispielsweise einen Polyether, einen Polyester, ein Polyamid, ein Polysiloxan, ein Polyacrylat oder ein Polymethacrylat, handeln. Erfindungswesentlich ist, daß die erfindinngsgemäßen Polymerisate I mindestens eine Seitengruppe der allgemeinen Formel IV enthalten

-X-A-F (IV)

worin die Variablen X, A und F die vorstehend bei den erfindungsgemäßen Monomeren I angegebenen Bedeutungen haben.

Außer der erfindungswesentlichen Seitengruppe der allgemeinen Formel IV können die erfindinngsgemäßen Polymerisate I noch mindestens eine Seitengruppe der allgemeinen Formel V enthalten.

-X-A-Y (V)

In der allgemeinen Formel V haben die Variablen X und A die vorstehend bei den erfindungsgemäßen Monomeren I angegebenen Bedeutungen. Dagegen steht die Variable Y für mindestens eine der folgenden Gruppen:

i) mesogene Gruppe, welche eine nematisch flüssigkristalline, eine smektisch flüssigkristalline, eine ferroelektrische smektisch flüssigkristalline und/oder cholesterisch flüssigkristalline Phase zu bilden vermag;

ii) nicht-zentrosymmetrische, ein leicht polarisierbares konjugiertes $\pi$-Elektronensystem sowie mindestens eine endständige Elektronenakzeptorgruppe enthaltende Gruppe und

iii) geradkettiger $C_{10}$- bis $C_{20}$-Alkylrest.

Seitengruppen V dieser Art sind üblich und bekannt und gehen zusammen mit ihrer Herstellung und Eigenschaften aus dem eingangs zitierten Stand der Technik hervor.

Wenn die erfindungsgemäßen Polymerisate I Seitengruppen der allgemeinen Formel V enthalten, dann können diese Seitengruppen V und die erfindungswesentlichen Seitengruppen der allgemeinen Formel IV in beliebigen Verhältnissen vorliegen. Sowohl die Auswahl der Seitengruppe der allgemeinen Formel V als auch deren Menge richtet sich vor allem nach dem Anwendungszweck der erfindungsgemäßen Polymerisate I und dem hierfür erforderlichen Eigenschaftsprofil:

Soll das erfindungsgemäße Polymerisat I beispielsweise nematisch flüssigkristalline Eigenschafen haben, wird man als Variable Y der allgemeinen Formel V eine an sich bekannte mesogene Gruppe wählen, welche eine nematisch flüssigkristalline Phase zu bilden vermag.

Soll das erfindungsgemäße Polymerisat I smektisch flüssigkristalline Eigenschaften haben, wird man als Variable Y der allgemeinen Formel V eine mesogene Gruppe verwenden, welche eine smektisch flüssigkristalline Phase zu bilden vermag.

Soll das erfindungsgemäße Polymerisat I ferroelektrische smektisch flüssigkristalline Eigenschaften haben, wird man als Variable Y in der allgemeinen Formel V eine mesogene Gruppe wählen, welche eine ferroelektrische smektisch flüssigkristalline Phase zu bilden vermag.

Soll dagegen das erfindungsgemäße Polymerisat I cholesterisch flüssigkristalline Eigenschaften haben, wird man als Variable Y der allgemeinen Formel V eine mesogene Gruppe wählen, welche eine cholesterisch flüssigkristalline Phase zu bilden vermag.

Soll das erfindungsgemäße Polymerisat I nichtlinear optische Eigenschaften haben, wird man als Variable Y in der allgemeinen Formel V eine nichtzentrosymmetrische, ein leicht polarisierbares konjugiertes $\pi$-Elektronensystem sowie mindestens eine endständige Elektronenakzeptor enthaltende Gruppe wählen.

Soll das erfindungsgemäße Polymerisat zu all diesen Eigenschaften oder anstelle von diesen auch noch für die Herstellung von Langmuir-Blodgett-Schichten geeignet sein, wird man als Variable Y der allgemeinen Formel V einen geradkettigen $C_{10}$- bis $C_{20}$-Alkylrest wählen.

Hierbei wird der Fachmann das Mengenverhältnis von obligatorischen Seitengruppen der allgemeinen Formel IV zu den fakultativen Seitengruppen der allgemeinen Formel V so einstellen, daß weder die vorteilhaften photochromen noch die wünschenswerten flüssigkristallinen und/oder nichtlinear optischen Eigenschaften und/oder diejenigen Eigenschaften, welche für die Langmuir-Blodgett-Schichtbildung verantwortlich sind, verloren gehen. So ist es beispielsweise im Falle von erfindungsgemäßen Polymerisaten I mit nematisch und/oder smektisch flüssigkristallinen Eigenschaften von Vorteil, wenn das Molverhältnis von Seitengruppen der allgemeinen Formel IV zu Seitengruppen der allgemeinen Formel V bei 0,01:99,99 bis 20:80 liegt.

Bei den besonders vorteilhaften erfindungsgemäßen Polymerisaten I handelt es sich um Acrylat- und Methacrylat-(Co)Polymerisate und um Organopolysiloxane. Von diesen sind die Acrylat- und Methacrylat-Copolymerisate ganz besonders vorteilhaft.

Beispiele ganz besonders vorteilhafter erfindungsgemäßer Polymerisate I sind die Polymerisate PI-1 und PI-2.

PI-1

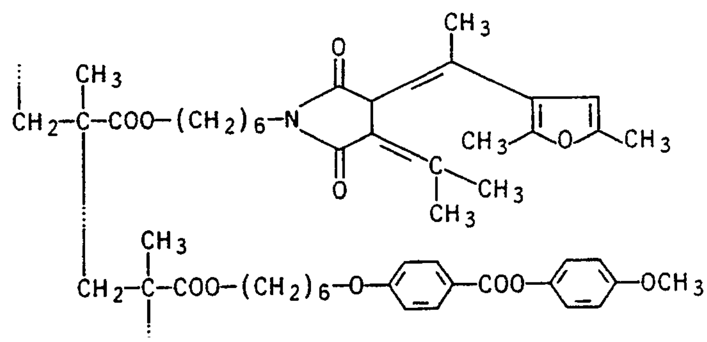

und die entsprechende cyclische Form;

PI-2

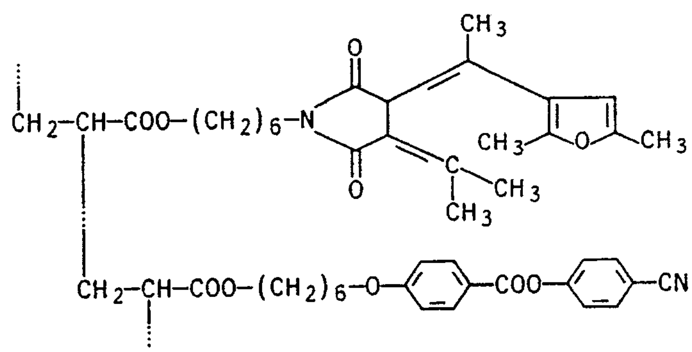

und die entsprechende cyclische Form.

Die erfindungsgemäßen Polymerisate I können nach Methoden hergestellt werden, wie sie in der Polymerenchemie üblich und bekannt sind.

Handelt es sich bei den neuen Polymerisaten I um Organopolysiloxane, dann können sie in besonders einfacher Weise durch die Addition mindestens eines der erfindungsgemäßen Monomeren I an Organopolysiloxane, welche mindestens eine SiH-Gruppe enthalten, hergestellt werden. Hierbei reagieren die betreffenden SiH-Gruppen sehr leicht mit den endständigen ethylenisch ungesättigten Gruppe der erfindinngsgemäßen Monomeren I unter Bildung von SiC-Einfachbindungen. Reaktionen dieser Art sind ebenso bekannt wie

die hierfür geeigneten Organopolysiloxane und gehen beispielsweise aus dem eingangs genannten Stand der Technik hervor.

Handelt es sich bei den erfindungsgemäßen Polymerisaten I um Acrylat- oder Methacrylat-(Co)-Polymerisate, können sie in üblicher und bekannter Weise durch radikalische (Co)Polymerisaten eines der erfindungsgemäßen Monomeren I, mehrerer erfindungsgemäßer Monomeren I untereinander oder mindestens eines erfindungsgemäßen Monomeren I mit mindestens einem an sich bekannten Monomeren, welches dem betreffenden erfindinngsgemäßen Copolymerisat I flüssigkristalline und/oder nichtlinear optische und/oder filmbildende Eigenschaften verleiht, hergestellt werden.

Schon bei ihrer Herstellung offenbaren sich die besonderen vorteilhaften Eigenschaften der erfindungsgemäßen Polymerisate I.

So sind sie in besonders einfacher Weise herzustellen, wobei ihre gewünschten Eigenschaftsprofile ganz gezielt und sicher eingestellt werden können. Dadurch ist es möglich, die neuen erfindungsgemäßen Polymerisate I für einen bestimmten Verwendungszweck maßzuschneidern, was ein Vorteil ist, der gar nicht hoch genug eingeschätzt werden kann. Außerdem können die erfindungsgemäßen Acrylat- oder Methacrylat-(Co)Polymerisate I aus den erfindungsgemäßen Monomeren I direkt auf strukturierten Oberflächen wie beispielsweise Orientierungsschichten aus angeriebenem Polyimid oder schräg aufgedampftem Siliciumdioxid in orientierter Form erzeugt werden. Neben den flüssigkristallinen, den nichtlinear optischen und/oder den filmbildenden Eigenschaften, welche sich über die Art und Menge der Seitengruppen der allgemeinen Formel V besonders einfach und elegant einstellen lassen, weisen die neuen Polymerisate I photochrome Eigenschaften auf. Hierbei lassen sie sich in besonders kurzer Zeit und mit hoher Quantenausbeute aus ihrer schwach gefärbten oder farblosen offenkettigen oder nicht-cyclischen Form durch ultraviolettes Licht in die entsprechende stark gefärbte cyclische Form umwandeln. Diese Form ist thermisch stabil und lagerfähig, so daß auch Informationen nach diesem Prinzip aufgezeichnet werden können. Die erfindungsgemäßen Polymerisate I in ihrer stark gefärbten, cyclischen Form können durch Bestrahlen mit sichtbarem Licht wieder leicht und mit hoher Quantenausbeute in die schwach gefärbte oder farblose nicht-cyclische Form umgewandelt werden. Dieser Vorgang kann mehrfach wiederholt werden, ohne daß es in einem störenden Ausmaß zu den Ermüdungserscheinungen kommt, welche häufig die Anwendung photochromer Verbindungen für die Datenaufzeichnung verhindern.

Die erfindungsgemäßen Polymerisate I eignen sich deshalb vorzüglich für die Holographie, wobei sie vor allem bei den holographischen Methoden angewandt werden können, wie sie von Christoph Bräuchle und Donald M. Burland in der Angewandten Chemie, Band 95 (1983), auf den Seiten 612 bis 629, beschrieben sind. Außerdem können die neuen Polymerisate I bei geeigneter Auswahl der Seitengruppen der allgemeinen Formel V die bislang bekannten Polymerisate in all den Anwendungszwecken ersetzen, wie sie von Donald R. Ulrich in dem Artikel "Non linear optical polymer systems and devices", in Molecular Chrystals and Liquid Chrystals, Band 160 (1988), Seiten 1 bis 31, beschrieben sind. Daneben haben die erfindungsgemäßen Polymerisate I bei geeigneter Wahl der Seitengruppen V hervorragende filmbildende Eigenschaften und können daher für die Herstellung von Langmuir-Blodgett-Schichten nach der Langmuir-Blodgett-Technik verwendet werden. Des weiteren können die erfindungsgemäßen Polymerisate I für die Herstellung von laseroptischen Datenplatten verwendet werden, wie sie beispielsweise in der GB-A-2 181 263, der US-A-4 752 820, der EP-A-0 205 187 oder der EP-A-0 171 045 beschrieben werden. Hierbei können die betreffenden laseroptischen Datenplatten auf der Basis der neuen Polymerisate I mit Hilfe der besonders vorteilhaften laseroptischen Schreib- und Leseverfahren der EP-A-0 278 446 beschrieben und gelesen werden.

Bei alledem ist noch zu bedenken, daß die erfindungsgemäßen Polymerisate I auch stets photochrome Eigenschaften haben. Hieraus ergeben sich neue Anwendungsmöglichkeiten für die erfindungsgemäßen Polymerisate I. So können sie in lichtempfindlichen Aufzeichnungselementen für die Reprographie verwendet werden. In diesen lichtempfindlichen Aufzeichnungselementen können sie in der lichtempfindlichen Aufzeichnungsschicht vorhanden sein, oder sie können einer sonstigen üblichen und bekannten lichtempfindlichen Aufzeichnungsschicht als Maskenschicht aufliegen. Bei der bildmäßigen Belichtung der Maskenschicht mit ultraviolettem Licht werden die bildmäßig belichteten Bereiche der Maskenschicht gefärbt und absorbieren daher sichtbares Licht sehr stark. Hiernach bestrahlt man das lichtempfindliche Aufzeichnungselement für die Reprographie mit sichtbarem Licht einer Wellenlänge, welche zwar eine photochemische Umwandlung der lichtempfindlichen Aufzeichnungsschicht aber keine Entfärbung der gefärbten Bereiche der Maskenschicht bewirkt. Hiernach kann das lichtempfindliche Aufzeichnungselement in üblicher und bekannter Weise entwickelt werden.

Darüber hinaus eignen sich die erfindungsgemäßen Polymerisate I für die Anzeige von Informationen auf beispielsweise großflächigen Bildschirmen. Durch ihren polymeren Aufbau und wegen ihrer photochromen Eigenschaften sind sie für diesen Anwendungszweck geradezu prädestiniert.

Wegen der vorstehend geschilderten besonderen Vorteile der erfindungsgemäßen Polymerisate I sind die Vorrichtungen für die Holographie, die laseroptischen Datenplatten, die lichtempfindlichen Aufzeichnungselemente für die Reprographie, die Vorrichtungen für die nichtlineare Optik, die Elemente zur Anzeige von Informationen und die Langmuir-Blodgett-Schichten auf der Basis der erfindungsgemäßen Polymerisate I den bislang bekannten Vorrichtungen, Datenplatten und Elementen überlegen.

Beispiele

Beispiel I
Die Herstellung und die Eigenschaften des Monomeren MI-1

Herstellvorschrift

Die Herstellung des erfindungsgemäßen Monomeren MI-1 geht aus von der Herstellung des betreffenden am Imidstickstoff unsubstituierten Fulgidimids.

Hierzu werden in einer ersten Stufe 1,5 g, entsprechend 5,76 mmol, des Fulgids Aberchrome E 540

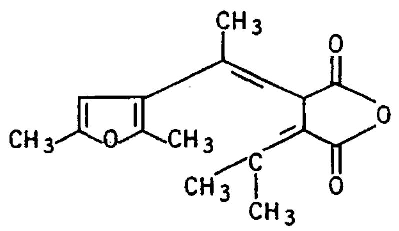

in 20 ml Aceton eingetragen. Nach der Zugabe von 2,1 ml 25 %iger Ammoniaklösung, entsprechend 27,8 mmol $NH_3$, wurde die resultierende Mischung 2 Stunden bei Raumtemperatur gerührt. Wie dünnschichtchromatographisch nachgewiesen werden konnte, war hiernach die Umsetzung des Fulgids zu der entsprechenden Amidsäure

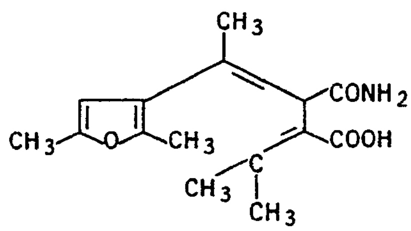

vollständig abgelaufen.

Nach dem Abdestillieren des Acetons und des Wassers resultierten 2,747 g der Amidsäure als orangegelbes Öl, welches in destilliertem Wasser gelöst wurde. Die resultierende Lösung hatte einen pH-Wert von 9,3. Die Lösung wurde ausgeethert und anschließend wurde sie mit 0,1 N Salzsäure bis pH 7 titriert. Die neutrale Lösung wurde eingedampft und der hierbei resultierende Rückstand wurde mit trockenem, analysenreinem Aceton aufgenommen, wobei sich Ammoniumchlorid als farbloser Niederschlag abschied. Hiernach enthielt die Acetonlösung nur noch die Amidsäure.

1,95 g dieser Amidsäure wurden in einer zweiten Stufe in analysenreinem Aceton gelöst und hiernach unter langsamen Rühren mit einer frisch hergestellten etherischen Diazomethanlösung im Überschuß versetzt. Hierbei konnte der Ablauf der Veresterungsreaktion an der Stickstoffentwicklung sehr gut verfolgt werden. Nach Beendigung der Reaktion wurde die Reaktionsmischung eingedampft. Hierbei wurden 1,728 g eines rotbraunen Rohprodukts erhalten, das mit Hilfe der Flash-Chromatographie mit einer 5 cm durchmessenden und 27 cm langen Säule und dem Laufmittel niedrigsiedender Petrolether/Aceton 2:1 gereinigt wurde. Hierbei wurde das Rohprodukt in Aceton auf die Säule aufgegeben.

Der hierdurch erhaltene reine Amidester

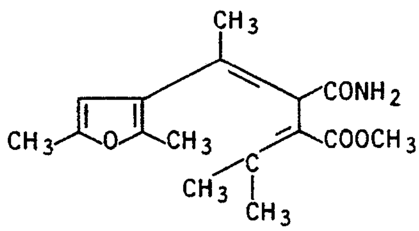

wurde dünnschichtchromatographisch charakterisiert ($R_f$-Wert: 0,88; Silicagel, Laufmittel Methylenchlorid/Methanol 6:1, Entwicklung mit einer Ce(IV)-Lösung).

Insgesamt wurden 1,375 g, entsprechend 4,72 mmol, des orangefarbenen Amidesters erhalten, was einer Ausbeute von 81,9 % der Theorie, bezogen auf das Ausgangsfulgid, entsprach.

In einer dritten Stufe wurden 1,36 g, entsprechend 4,67 mmol, des Amidesters in 12 ml heißem trockenen Tetrahydrofuran gelöst. Nach dem Erkalten der Lösung wurde hierzu eine ethanolische Natriummethanolat-Lösung hinzugegeben, welche 3,04 mmol $CH_3ONa \cdot 2CH_3OH$ enthielt. Die resultierende Reaktionsmischung war zunächst tiefrot gefärbt, indes entfärbte sie sich wieder nach kurzer Zeit. Die Reaktionsmischung wurde während 30 Minuten am Rückfluß gekocht und nach ihrem Erkalten auf ca. 200 ml Eis gegossen. Die hierbei resultierende klare orangefarbene Lösung wies einen pH-Wert von 14 auf. Sie wurde mit Hilfe von 1 N Salzsäure angesäuert und auf pH 5 eingestellt. Hiernach wurde die saure Lösung viermal mit Ether extrahiert. Die vereinigten Etherphasen wurden getrocknet und eingedampft. Hierbei wurden 1,095 g des Fulgidimids

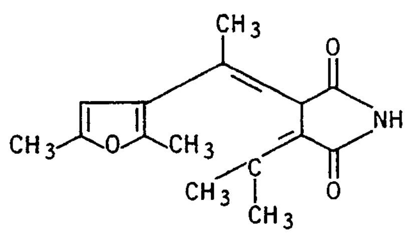

erhalten. Die Reinigung des Rohproduktes erfolgte mit Hilfe der Flash-Chromatographie an einer 5 cm durchmessenden und 16 cm langen Säule mit dem Laufmittel niedrigsiedender Petrolether/Essigsäureethylester 5:1. Es wurden 0,179 g, entsprechend 0,69 mmol, des Fulgidimids erhalten, was 14,8 % der Theorie entsprach. Das Fulgidimid kann dünnschichtchromatographisch sehr gut charakterisiert werden, weil es sich beim Bestrahlen mit ultraviolettem Licht einer Wellenlänge $\lambda$ von 366 nm rot färbt.

Die Stufen 1 bis 3 wurden in größeren Ansätzen beliebig oft wiederholt, um ausreichende Mengen des Fulgidimids für die Herstellung des erfindungsgemäßen Monomeren MI-1 zur Verfügung zu haben.

In einer vierten Stufe wurden 115 mg des Fulgidimids, entsprechend 0,438 mmol, in Tetrahydrofuran gelöst. Diese Lösung wurde zu einer Mischung von 108 mg 6-Hydroxyhexylacrylat, entsprechend 0,63 mmol, 157 mg Triphenylphosphin, entsprechend 0,59 mmol, und 108 mg Diazodicarbonsäurediethylester, entsprechend 0,59 mmol, sowie einer Spur p-Benzochinon als Polymerisationsinhibitor hinzugegeben. Die resultierende Reaktionsmischung wurde während 14 Stunden bei Raumtemperatur gerührt. Hierbei war das Reaktionsgefäß gegen sichtbares Licht geschützt. Die Reaktionsmischung wurde mit Hilfe der Flash-Chromatographie an einer 3 cm durchmessenden und 20 cm langen Säule mit dem Laufmittel niedrigsiedender Petrolether/Essigsäureethylester 10:1 durchgeführt, wobei das Reaktionsgemisch direkt in Tetrahydrofuran auf die Säule aufgegeben wurde. Die Fraktion, welche das erfindungsgemäße Monomere MI-1 enthielt, wurde mit Hilfe der Dünnschichtchromatographie charakterisiert, wobei zur Detektion eine ultraviolette Lampe verwendet wurde. Insgesamt wurden 74,2 mg 6-(N-Fulgidimid)hexylacrylat MI-1, entsprechend 0,179 mmol oder 40,4 % der Theorie, erhalten und kernresonanzspektroskopisch charakterisiert:

$^1H$-NMR ($CDCl_3$, Tetramethylsilan als interner Standard)

| chemische Verschiebung δ (ppm) | Signalform | Zuordnung |
|---|---|---|
| 1,28 | Singulett s | 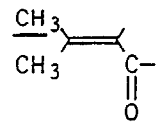 |
| 1,32-1,66 | Multiplett m | 8H, $-CH_2-$ |
| 1,94 | s | 2-$\underline{CH}_3$-Furan |
| 2,20 | s | 5-$\underline{CH}_3$-Furan |
| 2,28 | s | 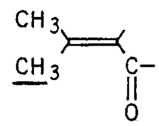 |
| 2,54 | s | 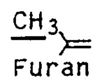 Furan |
| 3,55 | Triplett t | 2H, $-CH_2-$ |
| 4,13 | t | 2H, $-CH_2-$ |
| 5,77-5,80 | m | Acrylat-H |
| 5,87 | s | 1H, Furan-H |
| 6,04-6,12 | m | 1H, Acrylat-H |
| 6,34-6,38 | m | 1H, Acrylat-H |

Die Herstellung des erfindungsgemäßen Monomeren MI-1 wurde in größeren Ansätzen beliebig oft wiederholt, um ausreichende Mengen für die Herstellung der erfindungsgemäßen Polymerisate I zur Verfügung zu haben.

Beispiel 2

Die Herstellung des erfindungsgemäßen Monomeren MI-2

Herstellvorschrift

Das erfindungsgemäße Monomere MI-2 wurde wie in Beispiel 1 beschrieben hergestellt, nur daß anstelle des 6-Hydroxyhexylacrylats das 6-Hydroxyhexylmethacrylat verwendet wurde. Insgesamt wurden 74,0 mg des erfindungsgemäßen Monomeren MI-2 6-(N-Fulgidimid)-hexylmethacrylat, entsprechend 0,173 mmol oder 29,3 % der Theorie, erhalten. Seine offenkettige Form hatte auf Silicagel mit Petrolether/Essigsäureethylester 9:1 als Laufmittel einen $R_f$-Wert von 0,26. Die cyclische, farbige Form hat unter denselben Bedingungen einen $R_f$-Wert von 0,07. Auch das erfindungsgemäße Monomere MI-2 wurde kernresonanzspektroskopisch charakterisiert:

$^1H$-NMR ($CDCl_3$, Tetramethylsilan als interner Standard)

| chemische Verschiebung δ (ppm) | Signalform | Zuordnung |
|---|---|---|
| 1,28 | Singulett s | 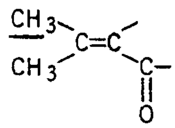 |
| 1,13-1,69 | Multiplett m | 8H, $-CH_2-$ |
| 1,91 | s | 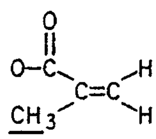 |
| 1,95 | s | 2-$\underline{CH}_3$-Furan |
| 2,21 | s | 5-$\underline{CH}_3$-Furan |
| 2,28 | s | 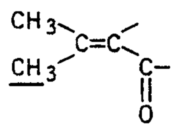 |
| 2,54 | s | 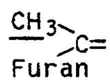 |
| 3,55 | Triplett t | 2H, $-CH_2-$ |
| 4,10 | t | 2H, $-CH_2-$ |
| 5,52 | s | 1H, Methacrylat-H |
| 5,88 | s | 1H, Furan-H |
| 6,06 | s | 1H, Methacrylat-H |

Die Herstellung des erfindungsgemäßen Monomeren MI-2 wurde in größeren Ansätzen beliebig oft wiederholt, um ausreichende Mengen für die Herstellung der erfindungsgemäßen Polymerisate I zur Verfügung zu haben.

Beispiele 3 bis 8 und Vergleichsversuche V1 und V2

Die Herstellung erfindungsgemäßer Polymerisate I (Beispiele 3 bis 8) und nicht erfindungsgemäßer Polymerisate (Vergleichsversuche V1 und V2)

Allgemeine Versuchsvorschrift

Sowohl die Polymerisation zu den nicht erfindungsgemäßen Polymerisaten (Vergleichsversuche V1 und V2) als auch die Copolymerisation zu den erfindungsgemäßen Polymerisaten I (Beispiele 3 bis 8) wurden in üblicher und bekannter Weise in Lösung durchgeführt, wobei Azobisisobutyronitril als Radikalinitiator verwendet wurde.

Das massenmittlere Molekulargewicht $\overline{M}w$ der hierbei erhaltenen erfindungsgemäßen Polymerisate I und der nicht erfindungsgemäßen Polymerisate wurde gelpermeationschromatographisch bestimmt.

Der Gehalt in Mol-% der erfindungsgemäßen Polymerisate I an Fulgidimideinheiten wurde durch kernresonanzspektroskopische Messungen bestimmt.

Die Phasenübergänge der erfindungsgemäßen Polymerisate I und der nicht erfindungsgemäßen Polymerisate wurden mit Hilfe der Differentialthermoanalyse (Differential Scanning Calorimetrie) bestimmt.

Die Tabelle 1 gibt einen Überblick über die Art und Menge der verwendeten Ausgangsverbindungen und über die Eigenschaften der erhaltenen erfindungsgemäßen Polymerisate I (Beispiele 3 bis 8) und der

nicht erfindungsgemäßen Polymerisate (Vergleichsversuche V1 und V2).

Wie aus der Tabelle 1 klar hervorgeht, weisen nur die erfindungsgemäßen Polymerisate I ein photochromes Verhalten auf. Außerdem konnte nachgewiesen werden, daß der Existenzbereich der flüssigkristallinen Phase über den Gehalt an Fulgidimidgruppen gezielt variiert werden kann.

Die in den Beispielen 3 bis 8 beschriebenen erfindungsgemäßen Polymerisate I konnten in der Holographie, der laseroptischen Datenaufzeichnung, der Reprographie, der nichtlinearen Optik und der Anzeige von Informationen verwendet werden.

Tabelle 1: Herstellung und Eigenschaften der erfindungsgemäßen Polymerisate I (Beispiele 3 bis 8) und der nicht erfindungsgemäßen Polymerisate (Vergleichsversuche V1 und V2)

| Beispiel Nr. | erfindungsgemäßes Monomer I (mol-%)[a] | bekanntes Monomer | zahlenmittleres Molekulargewicht $\bar{M}_w$[b] | Glastemperatur $T_G$[c] (°C) | Übergang von der flüssigkristallinen in die isotrope Phase[c] | Photochromie |
|---|---|---|---|---|---|---|
| 3 | MI-1 ( 4,24) | 1[d] | - | 20 | 97 | Ja |
| 4 | MI-1 ( 9,35) | 1[d] | - | 21 | 80 | Ja |
| 5 | MI-1 (14,12) | 1[d] | - | 22 | f) | Ja |
| 6 | MI-2 ( 2,61) | 2[e] | $2,6 \cdot 10^4$ | 42 | 98 | Ja |
| 7 | MI-2 ( 4,63) | 2[e] | $1,7 \cdot 10^5$ | 44 | 85 | Ja |
| 8 | MI-2 (11,11) | 2[e] | $1,7 \cdot 10^4$ | 44 | f) | Ja |
| Vgl.Vers. | | | | | | |
| V1 | | 1[d] | - | 20 | 105 | Nein |
| V2 | | 2[e] | $1,1 \cdot 10^5$ | 40 | 108 | Nein |

a) Bestimmung durch die Kernresonanzspektroskopie
b) Bestimmung durch die Gelpermeationschromatographie
c) Bestimmung durch die Differentialthermoanalyse
d) bekanntes Monomer 1:

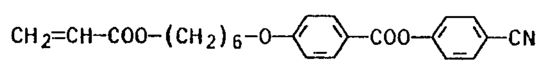

e) bekanntes Monomer 2:

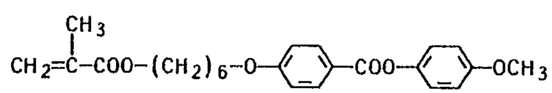

f) es tritt keine flüssigkristalline Phase mehr auf

## Patentansprüche

1. Monomere der allgemeinen Formel I

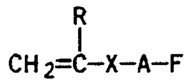 (I)

worin die Variablen die folgende Bedeutung haben:

R Wasserstoff- oder Chloratom oder Methylgruppe;

X zweiwertige verknüpfende funktionelle Gruppe oder Kohlenstoff-Kohlenstoff-Einfachbindung;

A flexible abstandshaltende langkettige Gruppe;

F über das Stickstoffatom mit A verbundene Fulgidimidgruppe.

**2.** Die Monomeren I nach Anspruch 1, dadurch gekennzeichnet, daß die Variable X die folgende Bedeutung hat:

X Sauerstoff- oder Schwefelatom,
Imidgruppe (-$NR^1$-), worin $R^1$ für ein Wasserstoffatom oder eine $C_1$- bis $C_4$-Alkylgruppe steht,
Estergruppe

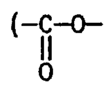 oder 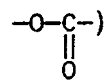 ,

Thioestergruppe

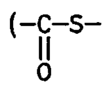 oder 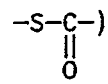 ,

Amidgruppe

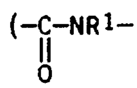 oder 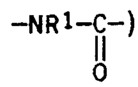 ,

worin $R^1$ die vorstehend angegebene Bedeutung hat, oder
Urethangruppe

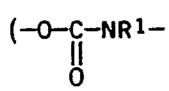 oder 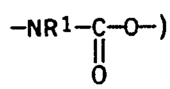 ,

worin $R^1$ die vorstehend angegebene Bedeutung hat.

**3.** Die Monomeren I nach Anspruch 1 oder 2, dadurch gekennzeichnet, daß die Variable R für ein Wasserstoffatom oder eine Methylgruppe steht.

**4.** Die Monomeren I nach einem der Ansprüche 1 bis 3, dadurch gekennzeichnet, daß die Variable X eine Estergruppe

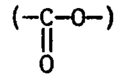

ist.

5. Die Monomeren I nach einem der Ansprüche 1 bis 4, dadurch gekennzeichnet, daß die flexible abstandshaltende langkettige Gruppe A die allgemeine Formel II hat,

$$-(CH_2)_k- \qquad (II)$$

worin der Index k eine ganze Zahl von 1 bis 20 bedeutet.

6. Verfahren zur Herstellung der Monomeren I gemäß einem der Ansprüche 1 bis 5, dadurch gekennzeichnet, daß man hierbei eine Verbindung der allgemeinen Formel III,

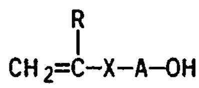

(III),

worin die Variablen R, X und A die vorstehend angegebenen Bedeutungen haben, mit einem an der Imidgruppe unsubstituierten Fulgidimid in der Gegenwart einer, bezogen auf das Fulgidimid, äquimolaren Menge an Triarylphosphin und einer katalytischen Menge eines Diazodicarbonsäuredialkylesters umsetzt und das hierbei gebildete Monomer I aus dem Reaktionsgemisch isoliert.

7. Verwendung der Monomeren I gemäß einem der Ansprüche 1 bis 5 und der gemäß Anspruch 6 hergestellten Monomeren I für die Herstellung photochromer und photochromer flüssigkristalliner Polymerisate.

8. Polymerisate I, welche mindestens eine Seitengruppe der allgemeinen Formel IV enthalten,

-X-A-F (IV)

worin die Variablen X, A und F die vorstehend angegebenen Bedeutungen haben.

9. Die Polymerisate I nach Anspruch 8, dadurch gekennzeichnet, daß sie mindestens eine Seitengruppe der allgemeinen Formel V enthalten,

-X-A-Y (V)

worin die Variablen X und A die vorstehend angegebenen Bedeutungen haben und worin die Variable Y für mindestens eine der folgenden Gruppen steht:

i) mesogene Gruppe, welche eine nematisch flüssigkristalline, eine smektisch flüssigkristalline, eine ferroelektrisch smektisch flüssigkristalline und/oder cholesterisch flüssigkristalline Phase zu bilden vermag;

ii) nicht-zentrosymmetrische, ein leicht polarisierbares konjugiertes $\pi$-Elektronensystem sowie mindestens eine endständige Elektronenakzeptorgruppe enthaltende Gruppe und

iii) geradkettiger $C_{10}$- bis $C_{20}$-Alkylrest.

10. Polymerisate I, herstellbar durch (Co)Polymerisation mindestens eines der Monomeren I gemäß einem der Ansprüche 1 bis 5 oder mindestens eines der gemäß Anspruch 6 hergestellten Monomeren I.

11. Polymerisate I, herstellbar durch Addition mindestens eines der Monomeren I gemäß einem der

Ansprüche 1 bis 5 oder mindestens eines der gemäß Anspruch 6 hergestellten Monomeren I an Organopolysiloxane, welche mindestens eine SiH-Gruppe enthalten.

**12.** Verwendung der Polymerisate I gemäß einem der Ansprüche 8 bis 11, der Monomeren I gemäß einem der Ansprüche 1 bis 5 sowie der gemäß Anspruch 6 hergestellten Monomeren I als photochrome Materialien.

**13.** Verwendung der Polymerisate I gemäß einem der Ansprüche 8 bis 11, der Monomeren I gemäß einem der Ansprüche 1 bis 5 sowie der gemäß Anspruch 6 hergestellten Monomeren I in der Holographie, der laseroptischen Datenaufzeichnung, der Reprographie, der nichtlinearen Optik und der Anzeige von Informationen.

**14.** Vorrichtungen für die Holographie, laseroptische Datenplatten, lichtempfindliche Aufzeichnungselemente für die Reprographie, Vorrichtungen für die nichtlineare Optik und Elemente zur Anzeige von Informationen, dadurch gekennzeichnet, daß sie mindestens eines der Polymerisate I gemäß einem der Ansprüche 8 bis 11 und/oder mindestens eines der Monomeren I gemäß einem der Ansprüche 1 bis 5 und/oder mindestens eines der gemäß Anspruch 6 hergestellten Monomeren I enthalten.

**15.** Verfahren zur Herstellung dünner orientierter photochromer Schichten, dadurch gekennzeichnet, daß man hierbei mindestens eines der Monomeren I gemäß einem der Ansprüche 1 bis 5 und/oder mindestens eines der gemäß Anspruch 6 hergestellten Monomeren I auf einer strukturierten Oberfläche (co)polymerisiert.

**16.** Verfahren zur Herstellung dünner orientierter photochromer Schichten, dadurch gekennzeichnet, daß man hierbei mindestens eines der Polymerisate I gemäß Anspruch 9, welche mindestens einen $C_{10}$- bis $C_{20}$-Alkylrest als Gruppe Y enthalten, mit Hilfe der Langmuir-Blodgett-Technik zu Langmuir-Blodgett-Schichten formt.